# EUROPÄISCHE PATENTSCHRIFT

(11) **EP 0 646 240 B1**
(45) Veröffentlichungstag und Bekanntmachung des Hinweises auf die Patenterteilung: **28.04.1999**
(21) Anmeldenummer: 93912390.7
(22) Anmeldetag: 08.06.1993
(51) Int. Cl.: G01N 33/00

(54) **VERFAHREN UND ANORDNUNG ZUR BEWERTUNG THERMISCHER UND DIELEKTRISCHER BEANSPRUCHUNG**
PROCESS AND ARRANGEMENT FOR ASSESSING THERMAL AND DIELECTRIC STRESS
PROCEDE ET DISPOSITIF PERMETTANT D'EVALUER LES CONTRAINTES THERMIQUES ET DIELECTRIQUES

(30) Priorität: 17.06.1992 AT 1232/92
(43) Veröffentlichungstag der Anmeldung: 05.04.1995
(73) Patentinhaber: ELIN ENERGIEVERSORGUNG GESELLSCHAFT m.b.H., 1141 Wien (AT)
(72) Erfinder: MÜLLER, Franz, D-8071 Grambach (AT)
(74) Vertreter: Rieberer, Stefan, Dipl.-Ing.
(86) Internationale Anmeldenummer: AT9300096
(87) Internationale Veröffentlichungsnummer: WO9325901

(56) Entgegenhaltungen:
- FR-A- 2 509 922
- GB-A- 2 059 584
- US-A- 3 978 732
- US-A- 4 117 713
- US-A- 4 130 009
- US-A- 4 436 699

## Beschreibung

Die Erfindung betrifft ein Verfahren zur Bewertung des thermischen und dielektrischen Zustandes einer elektrischen rotierenden Maschine bzw. einer elektrischen Anlage, welche Komponenten aus Isolierstoffen bzw. mit Überzugslacken aufweist, wobei die Maschine bzw. die Anlage mit einem geschlossenen Kühlkreislauf ausgerüstet ist und mittels eines gasförmigen Kühlmediums gekühlt wird, und wobei das gasförmige Kühlmedium nach der Abkühlung des Kühlmediums permanent analysiert wird. Weiters betrifft die vorliegende Erfindung eine Anordnung zur Durchführung des Verfahrens.

Die immer höher werdenden Maschinennutzungen, sowie die immer höher werdenden Anforderungen an die Betriebssicherheit rotierender Maschinen erfordern immer bessere Überwachungsmethoden. Ein wichtiger Teil ist die Überwachung der thermischen und auch der dielektrischen Belastung der Maschine, insbesondere des Wicklungssystems. Bei rechtzeitiger Feststellung von Veränderungen können geeignete Gegenmaßnahmen ergriffen werden und somit größere Schäden, wie zum Beispiel ein Brand, verhindert werden.

Derzeit erfolgt eine Überwachung von elektrischen Maschinen hinsichtlich der thermischen Beanspruchung von Maschinenkomponenten rotierender Maschinen, vorzugsweise elektrischer Wicklungssysteme, meistens durch Temperatursensoren, wie Thermoelemente oder temperaturabhängige Widerstände, welche im Bereich der Wicklung angeordnet sind. Bei Maschinen mit Hochspannungswicklungen wirkt sich vor allem der Umstand nachteilig aus, daß die Temperatursensoren nur dort angebracht werden können, wo durch geeignete, jedoch aufwendige, Maßnahmen eine dielektrische Beanspruchung der Sensoren vermieden wird. Aus Gründen der Betriebssicherheit und Zuverlässigkeit werden daher die Sensoren nur im Nutbereich der Wicklung, wo durch den Außenglimmschutz keine dielektrische Beanspruchung auftreten kann, angeordnet. Dies hat zur Folge, daß nur ein Bruchteil der gesamten Wicklung erfaßt werden kann.

Die in den letzten Jahren entwickelte Meßtechnik mit einer Datenübertragung über Lichtwellenleiter bietet zwar den Vorteil bei dielektrischen Belastungen einsetzbar zu sein, hat jedoch den Nachteil, daß diese Applikationen wegen der oft sehr schlechten Zugänglichkeit besonders gefährdeter Stellen nur bedingt angewendet werden können. Weiters sind auch diese Meßtechniken nicht in der Lage einen größeren Bereich der Wicklung zu überwachen als dies herkömmliche Sensoren können. Es ist daher nur eine punktuelle Überwachung möglich.

Aus der GB 2 059 584 A ist ein Verfahren bekannt, bei dem durch die gaschromatographische Analyse des auf Raumtemperatur abgekühlten Kühlgases auf eine etwaige Überhitzung der Maschine geschlossen werden kann. Unterschiedliche Kühlkreislaufabschnitte, die jeweils bestimmten Maschinenteilen zugeordnet sind, werden abwechseld der Analyse unterzogen um die überhitzte Stelle in der Maschine zu orten. Da die Zersetzung auf Überhitzung beruht, werden Partikelchen und Teilchen, also feste Körper frei, die im Gaschromatographen analysiert werden. Bei diesem bekannten Verfahren wird die durch Hitzeeinwirkung entstehende Destabilisierung von Materialverbunden bzw. Oberflächenstoffen erfaßt. Die Kohlenwasserstoffe treten erst bei sehr hohen Temperaturen auf, die weit über den Betriebstemperaturen und nahe von Brandausbrüchen liegen und sind ein Resultat, daß sich die Isolierstoffe zersetzen (beispielsweise bei einer Verkohlung der Oberfläche). Darüber hinaus müßen die Partikelchen und Teilchen mittels eines Trägergases transportiert werden. Als Trägergas kommt, durch die Verwendung eines Gaschromatographen, jedoch nur Helium, Neon, Argon oder, wie in den meisten Fällen, Wasserstoff in Frage. Diesem Verfahren haftet nun der Nachteil an, daß die Meßkammer jedesmal gespült werden muß und dieses Verfahren nur dann eingesetzt werden kann, wenn das Kühlgas gleich das Trägergas ist.

Auch aus der US-A-3 978 732 ist es bekannt, die Messung auf Partikelchen und Teilchen zu beziehen, welche zwar sehr klein sind und vom Trägergasstrom erfaßt werden. Jedoch handelt es sich auch in diesem Fall um Teilchen, da der Hinweis gegeben ist, daß sie zwar sehr klein, jedoch nicht so mobil wie Gasmolekühle sind. Darüber hinaus ist aufgezeigt, daß die Oberflächen mit organischen Substanzen bestrichen werden, die bei hohen Temperaturen sich von der Oberfläche abspalten. Im Sinne der Ortung von Lokalitätszonen werden verschiedene Substanzen verwendet.

Die Aufgabe der Erfindung besteht nun darin, ein zuverlässiges permanentes Verfahren zur Überwachung der Erwärmung und der dielektrischen Belastung von elektrischen Maschinen zu schaffen, welches die ganze Maschine bzw. Anlage erfaßt und das insbesondere zur Früherkennung von thermischen bzw. dielektrischen Beanspruchungen dient.

Die Aufgabe ist durch die Erfindung gelöst. Das erfindungsgemäße Verfahren ist dadurch gekennzeichnet, daß die gasförmigen Spaltprodukte und Zersetzungsprodukte und Ozon, hervorgerufen durch eine thermische bzw. dielektrische Beanspruchung der Isolierstoffe bzw. der Überzugslacke, bestimmt und gemessen werden, und daß diese Analysewerte bewertet werden, und der momentane Betriebszustand der Maschine bzw. der Anlage festgestellt wird, wobei die unterschiedliche Erwärmung bei unterschiedlichem Betriebszustand, wie etwa einem Teillastbetrieb, der Maschine bzw. Anlage berücksichtigt wird, sodaß eine thermische Überlastung erkannt werden kann, obwohl noch keine unzulässigen Übertemperaturen erreicht werden.

Mit dem erfindungsgemäßen Verfahren ist es erstmals möglich rotierende Maschinen permanent hinsichtlich ihrer thermischen und dielektrischen Beanspruchung zu überwachen, wobei die gesamte Maschine erfaßt ist. Bekanntlich setzen die verschiedenen Isolierstoffe bzw. Überzugslacke bei einer Erhöhung der thermischen Belastung aber auch bei dielektrischen Beanspruchung verschiedene Zersetzungsprodukte oder Spaltprodukte frei. Die Menge der freigesetzten Zersetzungsprodukte bzw. Spaltprodukte gibt Aufschluß über die Höhe der Belastung. Das jeweilige Zersetzungsprodukt bzw. Spaltprodukt gibt Aufschluß über die Lage der Überbeanspruchung innerhalb der Maschine. Die Anwesenheit von Ozon läßt jedoch auf eine hohe dielektrische Belastung wie z. B. Glimmen schließen. Da alle Komponenten vom Kühlmedium umströmt werden, sind die Spaltprodukte bzw. Zersetzungsprodukte der kompletten Maschine sowie das Ozon erfaßbar. Die Erfassung der gesamten Maschine bietet weiters den Vorteil, daß die unterschiedliche Erwärmung bei unterschiedlichem Betriebszustand der Maschine bzw. Anlage berücksichtigt ist. Somit wird auch bei einem Betriebszustand, wie etwa der Teillastbetrieb, eine thermische Überlastung der Maschine erkannt, obwohl hier noch keine unzulässigen Übertemperaturen erreicht werden. Mit dem erfindungsgemäßen Verfahren werden die zeitabhängigen Zustandsänderungen über die sich bildenden Spalt- und Zersetzungsprodukte beobachtet und zwar wenn noch keine unzulässigen Übertemperaturen erreicht werden.

Die Anordnung zur Durchführung des Verfahrens ist dadurch gekennzeichnet, daß das Kühlmedium dem geschlossenen Kühlkreislauf nach dem an sich bekannten Verfahren, bei dem das abgekühlte Kühlmedium, welches nach einem Maschinenkühler, der das aus der Maschine bzw. Anlage austretende erwärmte Kühlmedium kühlt, entnehmbar ist, und in ein Gasanalysegerät einspeisbar ist, und daß die Analyseergebnisse und Daten über den momentanen Betriebszustand der Maschine bzw. Anlage gemeinsam in ein Auswertegerät einspeisbar sind und das Auswertegerät mit einem Ausgabegerät verbunden ist.

Diese Anordnung bietet die Möglichkeit die gesamte Maschine bzw. Anlage sowohl hinsichtlich der thermischen als auch der dielektrischen Belastung permanent zu überwachen. Das abgekühlte gasförmige Kühlmedium wird mittels einem Gasanalysegerät auf das Vorhandensein und die Menge von Spaltprodukten der Isolierstoffe bzw. Überzugslacke permanent untersucht. Die Analyseergebnisse ermöglichen den Schluß auf eine thermische Beanspruchung - z.B. Höhe der auftretenden Temperaturen - und der dielektrischen Beanspruchung. Das Analyseergebnis wird dann in einem Auswertegerät mit den momentanen stationären oder instationären Betriebszuständen der Maschine bzw. Anlage gemeinsam bewertet.

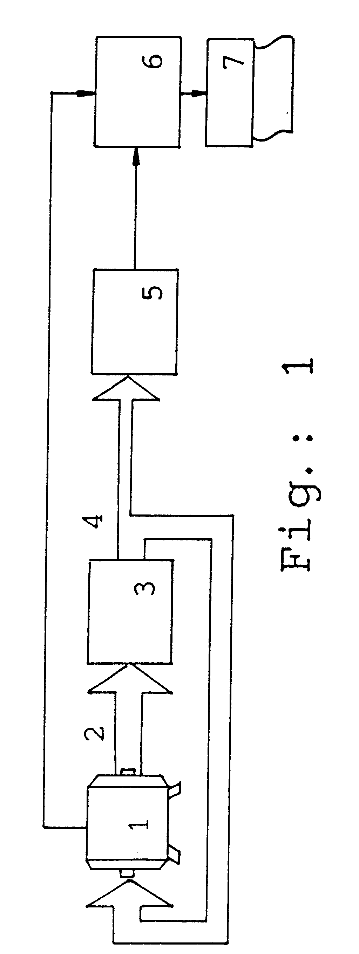

In der Folge wird die Erfindung anhand eines in der Zeichnung Fig. 1, in Form eines Blockschaltbildes, dargestellten Ausführungsbeispieles näher erläutert.

In Fig. 1 ist eine rotierende Maschine 1 ersichtlich, welche mit einem geschlossenen Kühlkreislauf ausgerüstet ist. Das von der Maschine 1 erwärmte Kühlmedium 2 wird in einem Kühler 3 abgekühlt und der Maschine 1 erneut zugeführt. Nach dem Kühler 3 wird eine geringe Menge des abgekühlten Kühlmediums 4 dem Analysegerät 5 zugeführt. Im Analysegerät 5 wird das abgekühlte Kühlmedium 4, auf das Vorhandensein und die Menge von Spaltprodukten bzw. Zersetzungsprodukten der Isolierstoffe bzw. Überzugslacke und Ozon, analysiert und die Daten in das Auswertegerät 6 eingespeist. Das Auswertegerät 6 wertet dann die Analysedaten und Daten über den Betriebszustand der Maschine - z.B. Teillastgemeinsam aus. Ergibt sich, aufgrund der analysierten Spaltprodukte bzw. Zersetzungsprodukte oder Ozon und deren Mengen, eine zu hohe thermische bzw. dielektrische Belastung bzw. Überlastung für den momentanen Betriebszustand der Maschine, so wird über ein Ausgabegerät 7 dies angezeigt, sodaß geeignete Gegenmaßnahmen ergriffen werden, um größere Schäden zu vermeiden. Auch ist eine ständige Protokollierung der thermischen bzw. dielektrischen Belastung möglich.

## Patentansprüche

1. Verfahren zur Bewertung des thermischen und dielektrischen Zustandes von einer elektrischen rotierenden Maschine bzw. einer elektrischen Anlage, welche Komponenten aus Isolierstoffen bzw. mit Überzugslacken aufweist, wobei die Maschine bzw. die Anlage mit einem geschlossenen Kühlkreislauf ausgerüstet ist und mittels eines gasförmigen Kühlmediums gekühlt wird, und wobei das gasförmige Kühlmedium nach der Abkühlung des Kühlmediums permanent analysiert wird, **dadurch gekennzeichnet**, daß die gasförmigen Spaltprodukte und Zersetzungsprodukte und Ozon, hervorgerufen durch eine thermische bzw. dielektrische Beanspruchung der Isolierstoffe bzw. Überzugslacke, bestimmt und gemessen werden, und daß diese Analysewerte bewertet werden, und der momentane Betriebszustand der Maschine (1) bzw. der Anlage festgestellt wird, wobei die unterschiedliche Erwärmung bei unterschiedlichem Betriebszustand, wie etwa ein Teillastbetrieb, der Maschine bzw. Anlage berücksichtigt wird, sodaß eine thermische Überlastung erkannt werden kann, obwohl noch keine unzulässigen Übertemperaturen erreicht werden.

2. Anordnung zur Durchführung des Verfahrens nach Anspruch 1, **gekennzeichnet dadurch**, daß das Kühlmedium dem geschlossenen Kühlkreislauf nach dem an sich bekannten Verfahren, bei dem das abgekühlte Kühlmedium (4), welches nach einem Maschinenkühler (3), der das aus der Maschine (1) bzw. Anlage austretende erwärmte Kühlmedium (2) kühlt, entnehmbar ist, und in ein Gasanalysegerät (5) einspeisbar ist, und daß die Analyseergebnisse und Daten über den momentanen Betriebszustand der Maschine (1) bzw. Anlage gemeinsam in ein Auswertegerät (6) einspeisbar sind und das Auswertegerät (6) mit einem Ausgabegerät (7) verbunden ist.

## Claims

1. Process for evaluating the thermal and dielectric condition of an electrical rotating machine or of an electrical installation that comprises components made of insulating materials or provided with coating varnishes, the machine or the installation being equipped with a closed cooling circuit and being cooled by means of a gaseous cooling medium, the gaseous cooling medium being permanently analysed after the cooling medium has cooled down, **characterised in that** the gaseous cleavage products and decomposition products and ozone, which are brought about by a thermal or dielectric stress of the insulating materials or coating varnishes, are determined and measured, and in that these analysis values are evaluated and the instantaneous operating condition of the machine (1) or of the installation is ascertained, whereby the different degree of heating for a different operating condition - such as an operation at partial load, for instance - of the machine or installation is taken into account, so that a thermal overload can be detected, even though no impermissible excess temperatures have yet been attained.

2. Arrangement for implementing the process according to Claim 1, characterised in that the cooling medium can be drawn from the closed cooling circuit in accordance with the process known as such, wherein the cooled cooling medium (4) which [sic] downstream of a machine cooler (3), which cools the heated cooling medium (2) emerging from the machine (1) or installation, and can be fed into a gas analyser (5), and in that the analytical results and data about the instantaneous operating condition of the machine (1) or installation can be jointly fed into an evaluating instrument (6), and the evaluating instrument (6) is connected to an output device (7).

## Revendications

1. Procédé permettant l'évaluation de l'état thermique et diélectrique d'une machine tournante électrique, respectivement d'une installation électrique, présentant des composants réalisés en matériaux isolants, respectivement avec des vernis de revêtement, la machine, respectivement l'installation étant équipée d'un circuit de refroidissement fermé et refroidie au moyen d'un milieu de refroidissement se présentant sous forme gazeuse, et où le milieu de refroidissement se présentant sous forme gazeuse est analysé en permanence après avoir procédé au refroidissement du milieu de refroidissement, caractérisé en ce que les produits de dédoublement et les produits de décomposition sous forme gazeuse et l'ozone, provoqués par une sollicitation thermique, respectivement diélectrique des matériaux isolants, respectivement des vernis de revêtement, font l'objet d'une détermination et d'une mesure et que ces valeurs d'analyse sont évaluées, et l'état de fonctionnement instantané de la machine (1), respectivement de l'installation est déterminé, la différence de chauffage en cas d'état de fonctionnement fluctuant, comme par exemple un fonctionnement en charge partiel de la machine ou de l'installation, étant pris en compte, si bien que peut être identifiée une surcharge thermique, bien qu'encore aucune surtempérature de valeur inadmissible n'ait été atteinte.

2. Dispositif de mise en oeuvre du procédé selon la revendication 1, caractérisé en ce que le milieu de refroidissement est susceptible d'être prélevé du circuit de refroidissement fermé, selon le procédé, connu en soi, dans lequel le prélèvement s'effectue sur le milieu de refroidissement (4), ayant été refroidi après passage dans un réfrigérant de machine (3) qui refroidit le milieu de refroidissement (2) ayant été chauffé et sortant de la machine (1), respectivement de l'installation, et ce milieu de refroidissement peut être injecté dans un appareil d'analyse de gaz (5), et en ce que les résultats d'analyse et les données concernant l'état de fonctionnement instantané de la machine (1), respectivement de l'installation, sont susceptibles d'être injectés conjointement dans un appareil d'évaluation (6), et l'appareil d'évaluation (6) est relié à un appareil d'édition (7).
